Europäisches Patentamt

(19) European Patent Office

Office européen des brevets

(11) Veröffentlichungsnummer: **0 222 852**
**B1**

(12) **EUROPÄISCHE PATENTSCHRIFT**

(45) Veröffentlichungstag der Patentschrifft:
07.02.90

(51) Int. Cl. [5]: **A 61 F 2/04**, A 61 M 25/00

(21) Anmeldenummer: 86903309.2

(22) Anmeldetag: 02.05.86

(86) Internationale Anmeldenummer:
PCT/EP 86/00263

(87) Internationale Veröffentlichungsnummer:
WO 86/06616 (20.11.86 Gazette 86/25)

(54) ENDOTUBUS.

(30) Priorität: 04.05.85 DE 8513185 U

(43) Veröffentlichungstag der Anmeldung:
27.05.87 Patentblatt 87/22

(45) Bekanntmachung des Hinweises auf die Patenterteilung:
07.02.90 Patentblatt 90/06

(84) Bennante Vertragsstaaten:
DE FR GB SE

(56) Entgegenhaltungen:
EP-A-0 138 089
FR-A-1 103 165
FR-A-2 232 334
GB-A-1 518 654
GB-A-2 069 339
US-A-4 043 338

(73) Patentinhaber: ESKA medical & kunststofftechnik gmbh
& co., vormals Walter Koss
Industriestrasse 2 Postfach 1164
D-6222 Geisenheim am Rhein (DE)

(72) Erfinder: Koss, Walter
Industriestrasse
D-6222 Geisenheim (DE)

(74) Vertreter: Blumbach Weser Bergen Kramer Zwirner
Hoffmann Patentanwälte
Sonnenberger Strasse 100
D-6200 Wiesbaden (DE)

EP 0 222 852 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

## Beschreibung

Die Erfindung betrifft einen Endotubus zum Plazieren insbesondere in der Speiseröhre mit einem rohrförmigen Mittelteil, an dessen einem Ende eine trichterförmige Erweiterung und am anderen Ende eine sich zum Mittelteil erweiternde Verdickung vorgesehen sind, die nach dem Durchtritt durch eine Verengung das Zurückziehen des Endotubus verhindert oder erschwert.

Es ist bekannt, bei Stenosen der Speiseröhre, die beispielsweise durch Karzinome bedingt sind, nach dem Bougieren einen Endotubus oder auch Überbrückungstubus einzusetzen, der dem Patienten die Möglichkeit verschafft, weiterhin feste und flüssige Speisen zu schlucken. Das Bougieren der Stenosen und später das Plazieren der Endotuben erfolgt in bekannter Weise entweder unter Röntgenkontrolle oder auch unter endoskopischer Sicht, wobei in bekannter Weise das Endoskop durch eine rohrförmige Öffnung des Bougies hindurchgesteckt wird. In gleicher Weise kann das Endoskop auch durch die Öffnung des Endotubus hindurchführen, so daß das Plazieren erleichtert wird. Damit der Endotubus nach dem Plazieren an seiner Stelle gesichert liegen bleibt, wird seine Vorderseite nach dem Plazieren entweder operativ vom Magen her festgelegt, oder der Endotubus erhält in bekannter Weise am vorderen Ende eine kragenförmige Verdickung, die das Zurückziehen durch die Stenose erschwert. Am hinteren (dem Mund zuweisenden) Ende sind die bekannten Endotuben mit trichterartigen Erweiterungen zur Anpassung an die anatomischen Verhältnisse und zur Lagesicherung versehen worden. Zu dem erläuterten Stand der Technik wird beispielsweise auf die EP-A-138 089 verwiesen. Weitere Beispiele von Endotuben sind erläutert in "British Journal of Surgery", Band 69 (1982), Seiten 61 bis 68, sowie auch in "Endoscopy" 8 (1976), Seiten 180 bis 185.

Aus der FR-A-2 232 334 ist eine Sonde bekannt, die unsymmetrisch nach vorn oder hinten geneigte Falten zur Verankerung der Sonde besitzt. Beim Einführen werden die Falten durch Strecken der Sonde zum Verschwinden gebracht.

Der Erfindung liegt die Aufgabe zugrunde, die bekannten Endotuben in Richtung auf ein einfacheres Plazieren auch unter endoskopischer Sicht sowie hinsichtlich ihrer Funktion und Handhabung zu verbessern.

Zur Lösung der Aufgabe geht die Erfindung aus von einem Endotubus der eingangs genannten Art und ist dadurch gekennzeichnet, daß die Verdickung aus zwei oder mehreren hohlen und zum Inneren des Mittelteils offenen, dünnwandigen Stegen besteht, die aus dem Mittelteil herausragen, deren Höhe in Richtung zum Mittelteil rampenartig ansteigt und die dann mit einem steilen Abfall enden, und daß die Stege vollständig eindrück- oder einfaltbar sind. Anders als die bekannten kragenartigen Erweiterungen können diese Stege auch dann, wenn ein Endoskop durch den Tubus geführt ist, vollständig eingedrückt oder eingefaltet werden, so daß keine Schwierigkeiten auftreten, wenn der Tubus durch eine Stenose eingeführt und plaziert wird. Nach dem Durchgang falten sich dann die Stege aus und verhindern dann aufgrund ihres steilen Abfalls, der zu einer Wirkung nach Art eines Widerhakens führt, daß der Tubus zurückgezogen werden kann.

Weiterbildungen der Erfindung sind Gegenstand der Unteransprüche. So weist zweckmäßig das rohrförmige Mittelteil im Anschluß an den steilen Abfall der Stege Abflachungen auf, die die Wandstärke des Mittelteils verringern. Dadurch wird das Einfalten der Stege ohne Durchmesservergrößerung des Endotubus weiter erleichtert. Die Bohrung des rohrförmigen Mittelteils wird zweckmäßig mit nach innen vorstehenden Längswülsten versehen, die vorzugsweise auch aneinander angrenzen können. Dadurch wird verhindert, daß beim späteren Abknicken eines in die Speiseröhre plazierten Tubus ein vollständiger Verschluß auftritt. Zwischen den abgeknickten Längswülsten bleibt immer ein wenigstens so großer Querschnitt bestehen, daß der Durchfluß von Speichel und die Aufnahme flüssiger Nahrung möglich ist.

In bekannter Weise kann in die Wand des rohrförmigen Mittelteils eine Versteifungswendel aus Kunststoff oder Stahldraht eingebettet sein. Der Widerstand des Tubus gegen Zusammendrücken im Bereich einer Stenose wird dadurch erhöht. Die trichterförmige Erweiterung besitzt mit Vorteil ovale Form in Anpassung an die anatomischen Verhältnisse. Wenn es später erforderlich werden sollte, einen Endotubus wieder zu entfernen, so kann dies dadurch erleichtert werden, daß in die Wand der trichterförmigen Erweiterung wenigstens ein Widerhaken eingebettet ist, der in das Innere der trichterförmigen Erweiterung hineinragt. Durch Einhängen eines Fadens oder Drahts in den Haken läßt sich dann der Endotubus wesentlich besser erfassen. Der Widerhaken kann aus Edelstahldraht gebogen oder ein Stanzteil aus Edelstahlblech sein.

Zum Einsetzen der Endotuben nach der Erfindung wird in bekannter Weise ein Plazierrohr verwendet, das ebenfalls auf das durch den Endotubus führende Endoskop aufgeschoben werden kann und in die trichterförmige Erweiterung unter Ausüben von Druck eingreift. In Verbindung hiermit empfiehlt eine Weiterbildung der Erfindung, daß die trichterförmige Erweiterung nach innen ragende, wulstartige Stege aufweist, die mit angepaßten Nuten am Fuß des Plazierrohrs eine drehfeste Verbindung bilden. Die ovale Ausführung der trichterförmigen Erweiterung genügt im allgemeinen nicht, um eine Verdrehung zwischen dem Tubus und dem Plazierrohr zu verhindern. Die Möglichkeit, den Tubus mittels des Plazierrohrs beim Einsetzen zu drehen, erleichtert aber häufig den Plaziervorgang. Die Widerhaken zum Ziehen eines Endotubus können zweckmäßig im Bereich der wulstartigen Stege in die Innenwand der trichterförmigen Erweiterung eingebettet sein. Dort stören die Haken am wenigsten, und außerdem erleichtert die größere Wandstärke im Bereich der wulstartigen Stege eine zugfeste Verankerung. Um die Herstellung der Verbindung zwischen Endotubus und Plazierrohr unter Röntgenkontrolle zu erleichtern, wird zweckmäßig in das Ende des Plazierrohrs ein Metallring eingebettet. Zur Anpassung an die individuellen Verhältnisse beim jeweiligen Patienten kann in Weiterbildung der Erfindung ein Ergänzungsstück auf die trichterförmige Erweiterung haftend aufgesetzt sein. Dabei ist das Ergänzungsstück so ausgebildet, daß es eine Verdickung und/oder Verlängerung der trichterförmigen Erweiterung bewirkt.

Schließlich besteht in weiterer Ausbildung der Erfindung die Möglichkeit, auf das rohrförmige Mittelteil wenigstens einen Erweiterungsring aufzustecken, der zweckmäßig dünn und hohl ausgebildet ist. Mit solchen Erweiterungsringen lassen sich beispielsweise Fisteln abdecken. Damit sich der Erweiterungsring beim Plazieren unter Entweichen oder Absaugen der enthaltenen Luft eng an den Tubus anlegen kann, kann vorgesehen sein, daß ein Schlauch in den hohlen Innenraum des Erweiterungsrings hineingeführt ist. Eine andere Möglichkeit besteht darin, an der Stelle des Erweiterungsrings die Wand des rohrförmigen Mittelteils zu durchbohren.

Der Endotubus sowie die Ergänzungsstücke und Erweiterungsringe werden mit Vorteil aus Silicongummi mit einer für medizinische Zwecke geeigneten Qualität oder auch aus anderen Elastomeren hergestellt.

Nachfolgend wird die Erfindung anhand von Ausführungsbeispielen in Verbindung mit den Zeichnungen beschrieben. Es zeigen:

Fig. 1  eine im vorderen und hinteren Bereich geschnittene Seitenansicht eines Endotubus nach der Erfindung;

Fig. 2  eine Seitenansicht des Tubus nach Fig. 1 in einer um 90° gedrehten Lage;

Fig. 3  die trichterförmige Erweiterung des Endotubus nach Fig. 1 und 2 mit zusätzlichen Stegen zur Verdrehungssicherung sowie den Kopf eines zugehörigen Plazierrohrs vor dem Einsetzen;

Fig. 4  eine Aufsicht des Endotubus nach Fig. 3 in Richtung auf die trichterförmige Erweiterung;

Fig. 5  perspektivisch einen aus Edelstahlblech gestanzten Widerhaken,

Fig. 6 und 7 teilweise geschnittene Seitenansichten des Endotubus nach Fig. 1 und 2 mit aufgesetzten Ergänzungsstücken;

Fig. 8  einen Ausschnitt des rohrförmigen Mittelteils eines Endotubus nach der Erfindung mit einem aufgeschobenen Erweiterungsring.

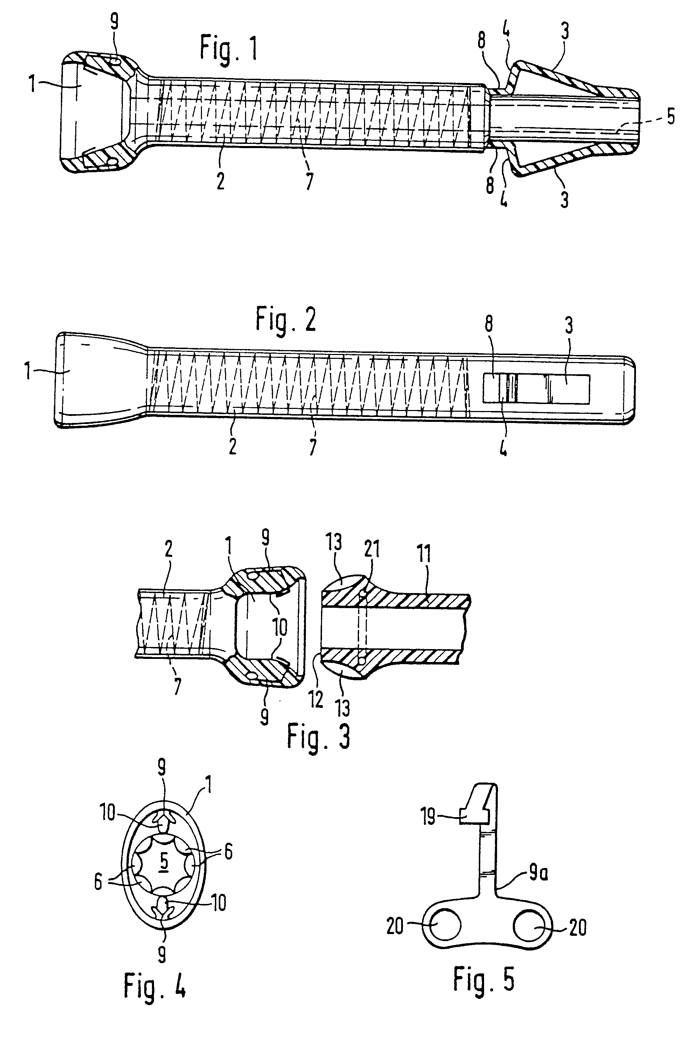

Der dargestellte Endotubus weist auf der in der Zeichnung linken Seite eine trichterartige Erweiterung 1 mit ovalem Querschnitt (vgl. Fig. 4) auf. Es schließt sich ein rohrförmiges Mittelstück 2 an, und am anderen Ende befinden sich auf zwei gegenüberliegenden Seiten hohle und dünnwandige, zum Inneren offene Stege 3, die rampenartig ansteigen und auf der zum Mittelteil 2 weisenden Rückseite einen steilen Abfall 4 besitzen. Durch den gesamten Endotubus führt eine Bohrung 5, mit der der Endotubus auf ein Endoskop (nicht gezeigt) zum Plazieren aufgesteckt werden kann. Die Bohrung 5 besitzt wenigstens im Bereich des Mittelteils 2 nach innen weisende Längswülste 6 (Fig. 4), die insgesamt einen gefurchten Querschnitt ergeben. Auch beim Abknicken bleibt dann ein restlicher Durchflußquerschnitt für Speichel und dünnflüssige Nahrung. In die Wand des Mittelteils 2 ist in an sich bekannter Weise eine Wendel 7 aus Stahldraht eingebettet, die eine Versteifung bewirkt.

Die hohlen Stege 3 können auch dann, wenn ein Endoskop durch die Bohrung 5 geführt ist, voll eingedrückt oder eingefaltet werden, so daß sie keine Durchmesservergrößerung bewirken und der Endotubus daher ohne zusätzliche Behinderung durch eine Stenose gedrückt und plaziert werden kann. Das Einfalten der Stege 3 wird durch eine Abflachung 8 des Mittelteils 3 im Anschluß an den Abfall 4 erleichtert.

In die Wand der trichterförmigen Erweiterung sind aus Stahldraht gebogene Haken 9 eingebettet, die in das Trichterinnere hineinragen. Es kann dann ein Faden oder ein Draht (nicht gezeigt) eingehängt werden, der das Ziehen eines plazierten Tubus erleichtert. Anstelle des aus Draht gebogenen Hakens 9 kann auch ein aus Edelstahlblech gestanzter Widerhaken 9a eingebettet sein, der in Fig. 5 gesondert dargestellt ist. Um ein Abrutschen des zum Ziehen benutzten Fadens oder Drahtes zu vermeiden, besitzt der Widerhaken 9a am freien Ende eine Erweiterung 19. Die Verankerung des Hakens 9a in der Wand des Endotubus wird durch Bohrungen 20 verbessert.

Bei dem abgewandelten Ausführungsbeispiel gemäß Fig. 3 und 4 ragen wulstartige Stege 10 in das Innere der trichterförmigen Erweiterung 1 hinein. Ein in Fig. 3 schematisch dargestelltes Plazierrohr 11 besitzt an seinem Fuß 12 an die Stege angepaßte Ausnehmungen 13. Wenn dann das Plazierrohr 11 in die trichterförmige Erweiterung 1 eingedrückt wird, ergibt sich schon aufgrund der ovalen Ausführung der Erweiterung 1 und zusätzlich durch die Stege 10 und Nuten 13 eine drehfeste Verbindung, derart, daß der Endotubus mit Hilfe des Plazierrohrs zur Erleichterung des Plaziervorgangs gedreht werden kann. Der Fuß des Plazierrohrs 12 und der Boden der trichterförmigen Erweiterung 1 um die Bohrung 5 herum sind so ausgebildet, daß sich eine großflächige Berührung ergibt, die eine gute Kraftübertragung ermöglicht, ohne daß sich jedoch der Endotubus und das Plazierrohr verkeilen. Nach dem Plazieren kann also das Plazierrohr 11 ohne Schwierigkeiten aus der trichterförmigen Erweiterung 1 herausgezogen werden. Zur Verbesserung der zugfesten Einbettung für die Stahldrahthaken sind diese im Bereich der Stege 10 angeordnet. Statt der Stahldrahthaken 9 können auch hier wieder gestanzte Widerhaken 9a gemäß Fig. 5 benutzt werden. In das Ende des Plazierrohrs 11 ist ein ringförmiger Metalldraht 21 eingebettet, der die Herstellung der Verbindung zwischen Endotubus und Plazierrohr 11 unter Röntgenkontrolle erleichtert.

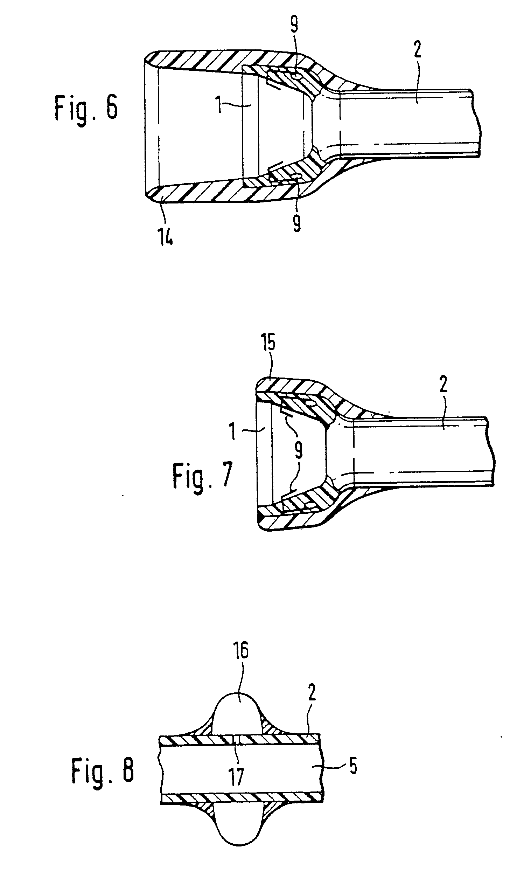

In Fig. 6 und 7 ist der Endotubus gemäß Fig. 1 und 2 im Bereich der trichterförmigen Erweiterung 1 mit einem Teil des rohrförmigen Mittelteils 2 (bei weggelassener Versteifungswendel 7) dargestellt, wobei jedoch auf die trichterförmige Erweiterung je ein Ergänzungsstück 14 bzw. 15 aufgesetzt sind. Bei richtiger Dimensionierung und gegebenenfalls Anfeuchten mit Alkohol entsteht nach dem Aufschieben des Ergänzungsstücks 14 bzw. 14 eine so gute Haftung, daß ein Abziehen praktisch nicht mehr möglich ist. Das Ergänzungsstück 15 bewirkt eine Vergrößerung der trichterförmigen Erweiterung 1, so daß sich je nach Ausführung des Ergänzungsstücks 15 jeweils gewünschte Anpassungen an die anatomischen Verhältnisse beim Patienten erzielen lassen. Auf ähnliche Weise kann mit dem Ergänzungsstück 14 eine Verlängerung des Endotubus erreicht werden. Das ist insbesondere auch zum Abdecken von Fisteln wichtig, die vermehrt nach Laserbestrahlungen auftreten. In Fig. 8 ist ein Abschnitt des rohrförmigen Mittelteils 2 des Endotubus nach den vorhergehenden Figuren dargestellt, wobei ein dünnwandiger Erweiterungsring 16 auf das Mittelstück 2 aufgeschoben ist. Der Erweiterungsring 16 ermöglicht beispielsweise ein Abdecken von Fisteln. Es können auch mehrere Ringe dieser Art je nach Bedarf vorgesehen sein. Damit der Ring 16 sich beim Plazieren des Endotubus eng anlegen kann, wird an der jeweiligen Stelle die Wand des Mittelteils 2 mit einer Bohrung 17 versehen. Dann kann die Luft aus dem Ring 16 beim Zusammendrücken entweichen und später nach dem Plazieren wieder eintreten. Zum gleichen Zweck kann man auch einen dünnen Schlauch (nicht gezeigt) in den Erweiterungsring 16 führen, der ein Entleeren bzw. Aufblasen des Rings ermöglicht.

**Patentansprüche**

1. Endotubus zum Plazieren insbesondere in der Speiseröhre mit einem rohrförmigen Mittelteil (2), an dessen einem Ende eine trichterförmige Erweiterung (1) und am anderen Ende eine sich zum Mittelteil erweiternde Verdickung vorgesehen sind, die nach Durchtritt durch eine Verengung das Zurückziehen des Endotubus verhindert oder erschwert, dadurch gekennzeichnet, daß die Verdickung aus zwei oder mehreren hohlen und zum Inneren des Mittelteils (2) offenen, dünnwandigen Stegen (3) besteht, die aus dem Mittelteil (2) herausragen, deren Höhe in Richtung zum Mitteilteil rampenartig ansteigt und die dann mit einem steilen Abfall (4) enden, und daß die Stege (3) vollständig eindrück- oder einfaltbar sind.

2. Endotubus nach Anspruch 1, dadurch gekennzeichnet, daß das rohrförmige Mittelteil (2) im Anschluß an den steilen Abfall (4) der Stege Abflachungen (8) aufweist, die die Wandstärke des Mittelteils verringern.

3. Endotubus nach Anspruch 1 oder 2, dadurch gekennzeichnet, daß die Bohrung (5) des rohrförmigen Mittelteils (2) nach innen vorstehende Längswülste (6) aufweist.

4. Endotubus nach Anspruch 3, dadurch gekennzeichnet, daß die Längswülste (6) aneinandergrenzen, derart, daß sich ein gefurchter Innenquerschnitt (Fig. 4) ergibt.

5. Endotubus nach Anspruch 1 bis 4, dadurch gekennzeichnet, daß in die Wand des rohrförmigen Mittelteils (2) eine Versteifungswendel (7) eingebettet ist.

6. Endotubus nach Anspruch 1 bis 6, dadurch gekennzeichnet, daß die trichterförmige Erweiterung (1) ovale Form besitzt.

7. Endotubus nach Anspruch 6, dadurch gekennzeichnet, daß in die Wand der trichterförmigen Erweiterung (1) wenigstens ein Widerhaken (9, 9a) eingebettet ist, der in das Innere der trichterförmigen Erweiterung (1) hineinragt und das Ziehen des Endotubus ermöglicht.

8. Endotubus nach Anspruch 7, dadurch gekennzeichnet, daß der Widerhaken (9) aus einem Edelstahldraht gebogen ist.

9. Endotubus nach Anspruch 7, dadurch gekennzeichnet, daß der Widerhaken (9a) ein Stanzteil aus Edelstahlblech ist.

10. Endotubus nach Anspruch 1 bis 9, dadurch gekennzeichnet, daß die trichterförmige Erweiterung (1) nach innen ragende, wulstartige Stege (10) aufweist.

11. Endotubus nach Anspruch 10, dadurch gekennzeichnet, daß die wulstartigen Stege (10) sich gegenüberliegend entlang der beiden Mantellinien verlaufen, in deren Bereich die Innenwand der trichterförmigen Erweiterung (1) den kleinsten Krümmungsradius besitzt.

12. Endotubus nach Anspruch 7 und 10 oder 11, dadurch gekennzeichnet, daß die Widerhaken (9, 9a) im Bereich der wulstartigen Stege (10) in die Innenwand der trichterförmigen Erweiterung (1) eingebettet sind.

13. Endotubus nach Anspruch 1 bis 12, dadurch gekennzeichnet, daß auf die trichterförmige Erweiterung (1) ein Ergänzungsstück (14, 15) haftend aufgesetzt ist, das eine Verdickung und/oder Verlängerung der trichterförmigen Erweiterung (1) bewirkt.

14. Endotubus nach Anspruch 1 bis 13, dadurch gekennzeichnet, daß auf das rohrförmige Mittelteil (2) wenigstens ein Erweiterungsring (16) aufgesteckt ist.

15. Endotubus nach Anspruch 14, dadurch gekennzeichnet, daß der Erweiterungsring (16) dünnwandig und hohl ausgebildet ist.

16. Endotubus nach Anspruch 15, dadurch gekennzeichnet, daß in den hohlen Innenraum ein Schlauch hineingeführt ist.

17. Endotubus nach einem der Ansprüche 10 bis 16 mit einem Plazierrohr (11), dadurch gekennzeichnet, daß am Ende (12) des Plazierrohrs (11) Nuten (13) oder Schlitze vorgesehen sind, die mit den wulstartigen Stegen (10) in der trichterförmigen Erweiterung (1) des Endotubus eine drehfeste Verbindung bilden.

18. Endotubus nach Anspruch 17, dadurch gekennzeichnet, daß am Ende des Plazierrohrs (11) ein Metallring (21) eingebettet ist.


**Claims**

1. Endotube for placement in the oesophagus in particular, with a tubular central portion (2), at one end of which is a funnel-shaped expanded portion (1) and at the other end of which is a thickened portion which expands towards the central portion, said thickened portion preventing or impeding the withdrawal of the endotube once it has passed through a narrow area, characterised in that the thickened portion consists of two or more hollow, thin-walled crosspieces (3) which are open towards the interior of the central portion (2) and which project from the central portion (2), the height of said crosspieces increasing in ramp fashion towards the central portion, and which then terminate in a steep downward slope (4), and in that the crosspieces (3) can be fully pressed or folded in.

2. Endotube according to claim 1, characterised in that the tubular central portion (2) has, following on from the steep downward slope (4) of the crosspieces, flattened portions (8) which reduce the wall thickness of the central portion.

3. Endotube according to claim 1 or 2, characterised in that the bore (5) of the tubular central portion (2) has inwardly protruding longitudinal beads (6).

4. Endotube according to claim 3, characterised in that the longitudinal beads (6) border on each another so as to produce a furrowed internal cross-section (Fig. 4).

5. Endotube according to claims 1 to 4, characterised in that a stiffening helix (7) is embedded in the wall of the tubular central portion (2).

6. Endotube according to claims 1 to 6, characterised in that the funnel-shaped expanded portion (1) is oval in shape.

7. Endotube according to claim 6, characterised in that at least one barb (9, 9a) is embedded in the wall of the funnel-shaped expanded portion (1), said barb protruding into the interior of the funnel-shaped expanded portion (1) and enabling the endotube to be removed.

8. Endotube according to claim 7, characterised in that the barb (9) is bent from a stainless steel wire.

9. Endotube according to claim 7, characterised in that the barb (9a) is a part punched from stainless steel sheet.

10. Endotube according to claims 1 to 9, characterised in that the funnel-shaped expanded portion (1) has inwardly protruding, bead-like crosspieces (10).

11. Endotube according to claim 10, characterised in that the bead-like crosspieces (10) extend opposingly along the two generating lines in the region of which the inside wall of the funnel-shaped expanded portion (1) has the smallest radius of curvature.

12. Endotube according to claims 7 and 10 or 11, characterised in that the barbs (9, 9a) are embedded in the region of the bead-like crosspieces (10) in the inside wall of the funnel-shaped expanded portion (1).

13. Endotube according to claims 1 to 12, characterised in that onto the funnel-shaped expanded portion (1) is adherently set a completion piece (14, 15) which produces a thickening and/or a lengthening of the funnel-shaped expanded portion (1).

14. Endotube according to claims 1 to 10, characterised in that at least one expansion ring (16) is set on the tubular central portion (2).

15. Endotube according to claim 14, characterised in that the expansion ring (16) is thin-walled and hollow.

16. Endotube according to claim 15, characterised in that a flexible tube is introduced into the hollow interior.

17. Endotube according to one of claims 10 to 16 with a placement tube (11), characterised in that at the end (12) of the placement tube (11) there are grooves (13) or slots which form a non-rotatable connection with the bead-like crosspieces (10) in the funnel-shaped expanded portion (1) of the endotube.

18. Endotube according to claim 17, characterised in that a metal ring (21) is embedded on the end of the placement tube (11).


**Revendications**

1. Endotube destiné à être placé dans l'oesophage notamment, comportant une pièce intermédiaire tubulaire (2) à une extrémité de laquelle est prévu un évasement (1) et à son autre extrémité un renforcement s'élargissant vers la pièce intermédiaire, qui empêche ou rend plus difficile le retrait de l'endotube après franchissement d'un étranglement, caractérisé en ce que le renforcement est constitué par deux ou plusieurs nervures creuses (3), à paroi mince, ouvertes vers l'intérieur de la pièce intermédiaire (2), qui dépassent celle-ci, dont la hauteur en direction de cette pièce intermédiaire s'élève en forme de rampe, pour se terminer ensuite par une retombée raide (4) et en ce que les nervures (3) sont totalement aplatissables ou repliables.

2. Endotube selon la revendication 1, caractérisé en ce que la pièce intermédiaire tubulaire (2) présente, après la retombée raide (4) des nervures, des méplats (8), qui diminuent l'épaisseur des parois de la pièce intermédiaire.

3. Endotube selon la revendication 1 ou 2, caractérisé en ce que l'alésage (5) de la pièce intermédiaire tubulaire (2) présente des bourrelets longitudinaux (6) faisant saillie vers l'intérieur.

4. Endotube selon la revendication 3, caractérisé en ce que les bourrelets longitudinaux (6) sont contigus de telle sorte qu'il en résulte une section transversale rainurée (figure 4).

5. Endotube selon les revendications 1 à 4, caractérisé en ce qu'une spirale de renforcement (7) est encastrée dans la paroi de la pièce intermédiaire tubulaire (2).

6. Endotube selon les revendications 1 à 5, caractérisé en ce que l'évasement (1) possède une forme ovale.

7. Endotube selon la revendication 6, caractérisé en ce qu'au moins un crochet (9, 9a) est encastré dans la paroi de l'évasement (1), crochet qui fait saillie à l'intérieur de l'évasement (1) et permet le retrait de l'endotube.

8. Endotube selon la revendication 7, caractérisé en ce que le crochet (9) consiste en un fil d'acier replié.

9. Endotube selon la revendication 7, caractérisé en ce que le crochet (9a) est une pièce en tôle d'acier spécial fabriquée à la presse.

10. Endotube selon les revendications 1 à 9, caractérisé en ce que l'évasement (1) présente des nervures (10) du type bourrelet, faisant saillie vers l'intérieur.

11. Endotube selon la revendication 10, caractérisé en ce que les nervures (10) de type bourrelet se font face le long de deux génératrices, dans le secteur desquelles la paroi intérieure de l'évasement (1) possède le plus petit rayon de courbure.

12. Endotube selon les revendications 7 et 10 ou 11, caractérisé en ce que les crochets (9, 9a) sont encastrés dans la paroi intérieure de l'évasement (1) dans le secteur des nervures (10) de type bourrelet.

13. Endotube selon les revendications 1 à 12, caractérisé en ce qu'une pièce complémentaire (14, 15) fixée sur l'évasement (1), sur lequel elle adhère, réalise un renforcement et/ou un allongement de l'évasement (1).

14. Endotube selon les revendications 1 à 13, caractérisé en ce qu'un anneau d'élargissement (16) au moins est rapporté sur la pièce intermédiaire tubulaire (2).

15. Endotube selon la revendication 14, caractérisé en ce que l'anneau d'élargissement (16) a une paroi mince et est creux.

16. Endotube selon la revendication 15, caractérisé en ce qu'un tuyau flexible est introduit dans l'espace interne creux.

17. Endotube selon l'une des revendications 10 à 16, comportant un tube de placement (11), caractérisé en ce qu'à l'extrémité (12) du tube de placement (11), on a prévu des évidements (13) ou des entailles, qui avec les nervures (10) de type bourrelet constituent dans l'évasement (1) de l'endotube une liaison résistant à la torsion.

18. Endotube selon la revendication 17, caractérisé en ce qu'à l'extrémité du tube de placement (11) est encastré un anneau métallique (21).

EP 0 222 852 B1

Fig. 1

Fig. 2

Fig. 3

Fig. 4

Fig. 5

Fig. 6

Fig. 7

Fig. 8